Europäisches Patentamt

European Patent Office

Office européen des brevets

(11) Publication number: **0 459 003 A1**

# EUROPEAN PATENT APPLICATION

(21) Application number: 90110476.0

(22) Date of filing: 01.06.90

(51) Int. Cl.⁵: **C09D 183/04**, C09D 5/14, A61L 9/01, A01N 59/16, A01N 25/08

(43) Date of publication of application:
04.12.91 Bulletin 91/49

(84) Designated Contracting States:
**DE FR GB**

(71) Applicant: **Kabushikikaisha Nippan Kenkyusho**
**17-3, Kanagawa 2-chome, Kanagawa-ku**
**Yokohama-shi, Kanagawa-ken(JP)**

(72) Inventor: **Ichikawa, Yoshio**
**6-49, Deguchi-cho**
**Chigasaki-shi, Kanagawa-ken(JP)**

(74) Representative: **Henkel, Feiler, Hänzel & Partner**
**Möhlstrasse 37**
**W-8000 München 80(DE)**

(54) Coating composition and antimicrobial deodorant composed of the coating compositions.

(57) A coating composition according to the present invention employs a hydrolytic silan compound (organoalkoxysilane) as a binder in order to increase the effective surface area of a coating with the use of ultrafine granular materials, so that the antimicrobial efficacy afforded by the catalytic action of metal ions of an antimicrobial metal, e.g silver, copper, and zinc. Also, during the development of the coating, the antimicrobial metal ions which are in solution state in the composition, are adsorbed and secured to not only a filler but also a hydrolizate of the silan compound acting as the binder, ensuring improvements in the antimicrobial and deodorant properties. Antimicrobial deodorants prepared by coating granular materials with the compositions which are then heated at a low temperature for a short time for curing, are excellent in the antimicrobial and deodorant properties and also, exhibits the higher resistance to water, hot water, acids, organic chemicals, climate, and wear.

EP 0 459 003 A1

## BACKGROUND OF THE INVENTION

The present invention relates to coating compositions and an antimicrobial deodorant composed of the coating compositions and more particularly, coating compositions for developing a coating, which has improved antimicrobial and deodorizing properties and ensures high resistance to water, chemicals, climate, heat, and the like, on a base material of metal, ceramic, plastic, wood, fiber, paper, etc and also, an antimicrobial deodorant which is unelusive and untoxic to human body, other creatures, and plant and tends to last long for preventing the propagation of unnecessary bacteria in agricultural and gardening soil so as to without the use of chemical fertilizers, promote the growth of plants, increase the harvest of crops, and allows the cultivation to be repeated, and for this purpose, adapted for inhibiting the propagation of unwanted bacteria and weeds in a water of water cultivation and also, of drink and cooking water containers, reservoirs, water vessels, bathes, pools, ponds, swamps, lakes, rivers, and the like particularly to eliminate the generation of odors, for avoiding the generation of mold when mixed in a cement material, for sterilizing an industrial water, and for eliminating odors from animal hats, sewerages, or sewage plants.

In prior arts, chlorine, nitrogen, or biochemical compounds are used for providing antimicrobial properties, which however, are less endurable resulting in the escape of toxic materials and also, may be dispersed and lessened in the efficacy.

Also, activated carbon, silica gel, zeolite, and the like are used for providing deodorant properties, which become less endurable when saturated and may be dispersed and declined in the inherent properties. When such a deodorant material is provided having a property of oxidative destruction (or antimicrobial property) and covered with a resin coating, the disadvantage is such that the effective surface area is reduced by the coating stress of a resin thus, degrading the chemical properties while wear causes a decrease in the effectiveness.

The present invention is directed, in view of the aforementioned problems existing in the prior arts, towards an unelusive, efficient, and inexpensive coating composition comprising a base material of, e.g. metal, ceramic, plastic, wood, fiber, paper, grain of sand or rubble, glass, shell, and baked clay, of which entire surface is covered with a coating carrying an antimicrobial metal, thus affording excellent antimicrobial and deodorant properties, ensuring high resistance to water, chemicals, and wear, and exhibiting improved durability and drying characteristics and also, towards an antimicrobial deodorant composed of the coating compositions.

## SUMMARY OF THE INVENTION

The present invention provides a coating composition comprising:

(a) 5 to 45 parts by weight of at least a material which is selected from the group consisting of organoalkoxysilane represented by the general formula, $R^1Si(OR^2)_3$ (where $R^1$ is an organic group having 1 to 8 carbon atoms and $R^2$ is an acyl group having 1 to 4 carbon atoms), hydrolyzate of the alkoxysilane, and partial condensates thereof;

(b) 0.3 to 40 parts by weight of at least a filler which is selected from the group consisting of silica gel, zeolite, activated carbon, ultrafine metallic oxide, and potassium titanate whisker;

(c) 0.03 to 3 parts by weight of an antimicrobial metal comprising at least a metal salt which is selected from the group consisting of silver, copper, and zinc salts;

(d) 3 to 60 parts by weight of water; and

(e) 5 to 80 parts of an alcohol, (where (a) + (b) + (c) + (d) + (e) = 100 parts by weight).

Also, the present invention provides an antimicrobial deodorant which is prepared by coating (f) a granular material with the coating composition which is in turn heated for curing.

## DESCRIPTION OF THE PREFERRED EMBODIMENTS

The present invention resides in the fact that the antimicrobial and deodorant efficacy of a coating composed of a composition carrying an antimicrobial metal is proportional to the surface area of the coating when the amount of the antimicrobial metal is constant and for the purpose of enhancement of the efficacy, employs easily processable and inexpensive granular matters to be coated, e.g. of sand, rubble stone, shell, baked clay, or the like.

The present invention is also disclosed in which a filler having an extensive specific surface area and carrying an antimicrobial metal adsorbed in ion state or built up by ion exchange is fixedly bonded to the surface of the granular material by an ultrafine gel layer of a hydrolyzate of organo-alkoxysilane which is excellent in the adhesion, durability, hardness, and resistance to water and chemicals.

The present invention will then be described with respect to the components of the composition.

(a) Organoalkoxysilane represented by the general formula, $R^1Si(OR^2)_3$, hydrolyzate of the alkoxysilane, and partial condensate thereof.

The organoalkoxysilane according to present invention is provided for stimulating hydrolyzation and polycondensation in the presence of water to constitute a polymeric form, a coating of which is readily cured at a normal temperature or by heating and thus, serves as a binder in the composition.

$R^1$ in the organoalkoxysilane is an organic group having 1 to 8 carbon atoms and may be an alkyl group (e.g. methyl group, ethyl group, n-propyl group, or i-propyl group), a $\gamma$-chloropropyl group, a vinyl group, a 3,3,3-trifluoropropyl group, a $\gamma$-glycidoxypropyl group, a $\gamma$-methacryloxypropyl group, a $\gamma$-mercaptopropyl group, a phenyl group, a 3,4-epoxycyclohexylethyl group, or a $\gamma$-aminopropyl group.

Also, $R^2$ in the organoalkoxysilane is either an alkyl group having 1 to 5 carbon atoms or an acyl group having 1 to 4 carbon atoms, e.g. a methyl group, an ethyl group, an n-propyl group, an i-propyl group, an n-butyl group, a sec-butyl group, a t-butyl group, and an acetyle group.

The organoalkoxysilane is available in the form of e.g. methyltrimethoxysilane, methyltriethoxysilane, ethyltrimethoxysilane, ethyltriethoxysilane, n-proyltrimethoxysilane, i-propyltrimethoxysilane, i-propyltriethoxysilane, $\gamma$-chloropropyltrimethoxysilane, $\gamma$-chloropropyltriethoxysilane, vinyltrimethoxysilane, vinyltriethoxysilane, 3,3,3-trifluoropropyltrimethoxysilane,3,3,3-trifluoropropyltriethoxysilane, $\gamma$-glycidoxypropyltrimethoxysilane, $\gamma$-glycidoxypropyltriethoxysilane, $\gamma$-methacryloxypropyltrimethoxysilane, $\gamma$-methacryloxypropyltriethoxysilane, $\gamma$-mercaptopropyltrimethoxysilane, $\gamma$-mercaptopropyltriethoxysilane, phenyltrimethoxysilane, phenyltriethoxysilane, $\gamma$-aminopropyltrimethoxysilane, 3,4-epoxycyclohexylethyltrimethoxysilane, or 3,4-epoxycyclohexylethyltriethoxysilane.

Methyltrimethoxysilane is most preferred as the organoalkoxysilane.

The organoalkoxysilane may also be prepared from one or more of those materials.

In action, the organoalkoxysilane liberates alcohol by hydrolyzation in an acid water medium, thus generating a corresponding silanol and simultaneously, stimulates the polycondensation for forming an organopolysiloxane compound.

The component (a) contains less than 50% by weight of at least a material which is selected from the group consisting of (a)' tetra-alkoxysilane represented by the general formula, $Si(OR')_4$ (where R' is a hydrocarbon residue having 1 to 5 carbon atoms), hydrolizate of the tetra-alkoxysilane, and partial condensate thereof.

The terta-alkoxysilane is available in the form of tetramethoxysilane, tetraethoxysilane, or tetra-n-butoxysilane.

The amount of the component (a) in the composition is generally 5 to 45 parts by weight; preferably, 15 to 30 parts by weight (where (a) + (b) + (c) + (d) + (e) = 100 parts by weight, throughout the specification and claims).

If less than 5 parts by weight, the adhesion of a coating will be declined and the hardness of the same will remain unimproved. Adversely, if more than 45 parts by weight, the coating will bear a flaw or crack and the lasting stability of the composition will be reduced.

(b) Filler

The filler (b) is provided for adsorption of the antimicrobial metal in ion state or ion exchange of the same and selected from the group consisting of silica gel, zeolite, activated carbon, ultrafine granular metallic oxide having a particle size of 5 to 100 millimicrons in average diameter, and potassium titanate whisker. The filler according to the present invention will now be described with respect to the components.

(Silicagel)

Silica gel is a compound expressed by the general formula, $SiO_2 \cdot nH_2O$, and found in a semitransparent granular form of which structure exhibits a coarse texture and has a high area/weight ratio of e.g. more than 450 $m^2$ to 1 g.

Preferably, the silica gel of the present invention is less than 10 micrometers in average diameter to ensure the development of a thin film having improved resistance to wear.

(Zeolite)

3

The zeolite according to the present invention is either a natural or synthetic zeolite expressed by the general formula,

$$xM_{2/n}O \cdot Al_2O_3 \cdot ySiO_2 \cdot zH_2O$$

(where M is an ion-exchangeable metallic ion of commonly mono- or bi-valent metal, n a valence number thereof, x a coefficient of the metallic oxide, y a coefficient of silica, and z the number of waters for crystallization), and available in various kinds which are different in composite ratio, diameter of pore, and specific surface ratio.

For example, the natural zeolites include analcime, chabasite, clinoptilolite, erionite, forgeasite, and mordenite and the synthetic zeolites include A-zeolite, X-zeolite, Y-zeolite, and T-zeolite.

The zeolite is preferably in granular form having a diameter of less than 10 micrometers. More preferably, each particle of the zeolite is less than 3 micrometers in diameter, its area/weight ratio is less than $150m^2:1g$, and the molar ratio of $SiO_2/Al_2O_3$ is less than 14:1.

The filler of the present invention is a highly adsorbate zeolite having a greater specific surface area; either a natural mordenite or a synthetic zeolite.

(Activated carbon)

Activated carbon is a black granular material prepared by carbonizing an organic matter, having a highly porous structure and thus, ensuring a power of adsorption.

The diameter of an activated carbon particle is a generally 0.5 to 10 micrometers; preferably, 1 to 3 micrometers, and the area/weight rate is more than 600 to 1,000 $m^2/g$.

The activated carbon is available in the form of charcoal, charred coconut, fossil charcoal, bone charcoal, animal charcoal, etc.

(Ultrafine granular metallic oxide)

The ultrafine granular metallic oxide according to the present invention contains colloidal silica prepared by dispersing ultrafine particles of silicic anhydride in a dispersing medium of water or alcohol, colloidal alumina or alumina sol with a dispersing medium of water, ultrafine granular silica prepared by high-temperature hydrolyzation of purified metal salt, ultrafine granular alumina, and ultrafine granular titanium. For development of a coating having a high area/weight ratio, the average diameter of the particles is preferably 5 to 100 millimicrons.

Also, the ultrafine granular metallic oxide may include an aqueous colloidal metallic oxide having a pH of 2 to 9 for maintaining the pH scale of the composition to an acidic level.

(Potassium titanate whisker)

The potassium titanate whisker according to the present invention is a single crystal fiber having the general formula, $K_2O \cdot nTiO_2$, and of which average length, average diameter, and volumetic specific gravity are 10 to 50 micrometers, 0.05 to 0.5 micrometers, and less than 0.3 respectively.

The potassium titanate whisker is adopted, as well as the ultrafine granular metallic oxide, for adsorption of the antimicrobial metal (c) with a greater extension of the specific surface area.

The amount of the filler (b) in the composition of the present invention is substantially, on the basis of solid phase, 0.3 to 40 parts by weight and more preferably, 2 to 20 parts by weight.

If the filler (b) is less than 0.3 parts by weight, the antimicrobial metal (c) will be less adsorbed and simultaneously, the ion exchange will become deficient causing elusion. Also, if more than 40 parts by weight, the composition itself is increased in viscosity and the hardness of its coating will be reduced,

(c) Antimicrobial metal

The antimicrobial metal (c) is provided for affording antimicrobial properties against microbes such as bacteria, funguses, water weeds, or the like and acting as an oxidizer for various odors. Also, the antimicrobial metal (c) is a metal salt, serving as an acidic catalyst for hydrolyzation of the organoalkoxysilane contained in the component (a).

The antimicrobial metal (c) is carried in ion form by the coating containing the filler (b) and as known, its antimicrobial and oxidizing functions are stimulated by activated oxygen which is acquired from a portion

EP 0 459 003 A1

of oxygen in the air or the remaining oxygen in water by the catalytic action of metal ions.

Because non of silver and copper is detected in an immersion water, it is assumed that the catalytic function is present. Hence, the antimicrobial and deodorant activities of the antimicrobial deodorant of the present invention will last for a considerable period of time.

The antimicrobial metal (c) is carried not only by the filler but also by the ultrafine granular gel of the organoalkoxysilane which is the component (a) acting as the binder and thus, the antimicrobial coating of the present invention will exhibit higher powers of both antimicrobial activity and oxidization.

the antimicrobial metal (c) is a metal salt of silver, copper, or zinc which is available in, but not limited to, the form of silver nitrate, silver sulfate, silver chloride, copper nitrate (II), copper sulfate, copper bromide, copper acetate, zinc nitrate (II), zinc perchlorate, etc. It is understood that one or more of the metal salts are dissolved in a water for use as a mixed solution.

The mixed water solution permits the filler (b) in the composition to activate adsorption and ion exchange.

The amount of the antimicrobial metal (c) in the composition of the present invention is preferably 0.03 to 3 parts by weight and more preferably, 0.1 to 1.5 parts by weight. If the antimicrobial metal (c) is less than 0.03 weight part, the antimicrobial efficacy which is a primary object of the present invention will remain inactive. If more than 3 parts by weight, the elusion will occur.

(d) Water

The water (d) is an essential component for hydrolyzation of the organoalkoxysilane of the component (a) and also, serves as a dispersion medium for the filler (b).

The water is common service water, distilled water, or ion exchange water. Particularly, when the composition needs to be highly purified for preparing an antimicrobial deodorant, either the distilled or ion exchange water is preferred. It would be understood that the waters (d) include a water generated during the hydrolyzation of the organoalkoxysilane of the component (a) and a water contained, in the form of aqueous colloidal alumina, in the filler (b).

The amount of the water in the composition of the present invention is commonly 3 to 60 parts by weight and preferably, 10 to 40 parts by weight. If less than 3 parts by weight, the hydrolyzation of the organoalkoxysilane of the component (a) will be discouraged and if over 60 parts by weight, the composition of the present invention will be declined in the stability.

(e) Alcohol

The alcohol is provided for acting as a dispersion medium for the filler (b) and the antimicrobial metal (c), preventing the excessive gelation of the organoalkoxysilane of the component (a) during the hydrolyzation with water, and removing a water by azeotropic distillation with the control over the polycondensing action of the hydrolyzate.

The alcohol (e) may be a monohydric alcohol, an ethylene glycol of dihydric alcohol, or derivative thereof. The monohydric alcohol is preferably a low aliphatic alcohol having 1 to 5 carbon atoms and available in the form of methanol, ethanol, n-propyl alcohol, i-propyl alcohol, sec-butyl alcohol, t-butyl alcohol, etc. The ethylene glycol and its derivative are available in the form of ethylene glycol, ethyleneglycol monobutylether, ethyleneglycol monoethylether acetate, etc.

Preferably, the alcohols (e) include i-propyl alcohol, sec-butyl alcohol, ethyleneglycol monoethylether acetate, and mixtures of more than one thereof.

The amount of the alcohol in the composition of the present invention is commonly 5 to 80 parts by weight and preferably, 15 to 40 parts by weight. If less than 5 parts by weight, the composition will be separated into two layers or gelatinized. If more than 80 parts by weight, the other components relatively decrease in the composition and thus, the resultant coating will be declined in the bonding to the base material and also, become too thin to produce a desired antimicrobial deodorant.

For developing the coating carrying the antimicrobial metal, the coating composition according to the present invention contains the foregoing components (a), (b), (c), (d), and (e) and if necessary, may include an inorganic pigment, a surface-active agent, a coupling agent, an acid, an alkali metal salt, a known additive, and the like.

For preparation of the composition of the present invention, the processing of the components is carried out, but not limited to, by dissolving (c) into (d), activating (b) for either adsorption or ion exchange, and then, supplying (e) and (a) in sequence or by dissolving (c) into (d), supplying (e) and (b) in an order for either adsorption or ion exchange, and finally, supplying (a).

5

The coating composition according to the present invention is prepared by a sequence of mixing of (c) → (d) → (b) → (e) → (a).

After the mixing, the component is preferably left for maturing for a duration of 2 to 72 hours and more preferably, 3 to 24 hours.

If the maturing duration is less than 2 hours, the hydralyzation and polycondensation of the component (a) will remain incomplete failing to eliminate the heat of reaction and causing the coating to be hostile to the base material.

If more than 70 hours, the polycondensation of the component (a) will proceed too fast causing a decline in the dispersing stability of the composition and also, a reduction in the density, bonding, and hardness of the coating which thus appears less glossy.

Preferably, the maturing is commonly carried out at a normal temperature of 5 to 30° C.

The coating composition of the present invention can be agitated for dispersion by a high-speed stirrer, a ball mill, or the like dispersion mixer and then, filtered to a uniform and stable dispersion liquid.

(f) Granular material

The granular materials employed according to the present invention are provided for serving as a base onto which the coating composition is applied for heating and curing, thus constituting an antimicrobial deodorant.

Preferably, the material is highly durable and inexpensive and has a greater extension of the specific surface area for permitting optimum antimicrobial and deodorant activity of the antimicrobial deodorant.

The granular material (f) is available in the form of sand, rubble, shell, glass, or inorganic baked clay material.

The sands include silica, lime, river sand, sea sand, mountain sand, and other natural sandy matters.

The rubble stones include bits of artificially broken stone or rock, and a mineral residue remaining after a process of e.g. iron manufacturing.

The shells include grains of natural shells, e.g oyster, scallop, and wreath shells, and of calcific coral.

The glass consists of beads of glass.

The inorganic baked clay material is prepared by baking at 500 to 1,500° C a clay mineral matter, e.g. kaolin, plastic clay, frog-eye clay, clay soil, bentonite, or a mixture of the clay matter and a relating mineral matter, e.g. quartz, feldspar, alumina, mulite, wollastonite, and talc, to a solid which is thus light weighted and highly porous.

The average diameter of the granular material is 0.1 to 30 mm and preferably, 0.5 to 10 mm. If smaller than 0.1 mm, the material becomes scatterable and elusive. If greater than 30 mm, the action of dispersion will be discouraged and also, the specific surface area will relatively decrease.

The antimicrobial deodorant of the present invention is thus formed by applying the coating composition containing the antimicrobial metal onto the granular material and heating the same for curing. The coating composition is preferably 0.2 to 20 % (when dried out) of the weight of the granular material and more preferably, 0.5 to 5 %.

The coating of the coating composition is carried out by applying the composition prepared in the aforementioned manner onto the granular material by a coating means, e.g. dipping, mixing, spraying, and daubing and then, heating the same for curing at 100 to 300° C for 5 to 60 minutes until a coating layer which is improved not only in the antimicrobial and deodorant properties but also in the resistance to water, chemicals, and wear is generated.

It is assumed that the antimicrobial deodorant of the present invention exhibits an antimicrobial property when applied 0.1 to 5 % by weight into a water in a tub and after three years, no change in color and smell nor generation of water weeds will be detected. In a practical test for the antimicrobial property of such an antimicrobial water which was conducted after three years of usage, the activity of the antimicrobial property was found even higher than 3 years ago. It is thus understood that the antimicrobial deodorant of the present invention when used in a water turns the water to a sterilized water and maintains it for a long period of time.

Also, the antimicrobial deodorant of the present invention exhibits a higher power of sterilization in a nutritious water and thus, will be used with equal success for eliminating odors from animal hats, sewer systems, rotten creatures, and the like.

The antimicrobial deodorant of the present invention when sprayed 5 to 200 grams per square meter in the ground can sterilize soils which then maintains its sterilizing power for a long time.

Hence, non-sterilizer cultivation will be possible. In fact, using the antimicrobial deodorant involves the higher growth of agricultural products, fruits, flowers, and other greens. Particularly, through the results of various tests, the harvest of rice grains, vegetables, strawberries, and grapes was found a increase of 20 to 60%, and also, the increase of sugar in strawberries and grapes was detected.

As set forth above, the present invention provides antimicrobial deodorants which are improved in the antimicrobial and deodorant properties, thus preventing the propagation of unwanted bacteria in soils for agriculture and gardening, ensuring the rapid growth and incremental harvest of agricultural products, fruits, flowers, lawn greens, and the like or the repeated cultivation of the agricultural products, and sterilizing a water for water cultivation to eliminate the propagation of undesired bacteria and the generation of water weeds. This allows no use of agricultural sterilizers or a considerable reduction of its use.

The antimicrobial deodorants when used in water offer a higher antimicrobial activity, thus sterilizing the water in reservoir containers, bath tubs, or pools and of ponds, lakes, or rivers for an extensive period of time and also, providing long lasting antimicrobial or sterilized water for beverage and cooking and also, for food, cosmetic, and other industrial use. They can also be employed in water or air filters and water treatment systems and furthermore, applicable to eliminate odors from houses of living creatures.

Particularly, the antimicrobial deodorants of the present invention which are excellent in the antimicrobial and deodorant properties, are also improved using in the resistance to heat, climate, hot water, acids, and organic chemicals, the thermal dissipation, and the hardness (resistance to wear) and yet, low in the cost of production and can be handled with ease.

The advantages of the present invention will be utilized for livelihood, industrial, agricultural, fishery, medical, and other applications and thus, widely appreciated in industrial concerns.

The embodiments of the present invention will now be described in more details referring to Examples. The present invention is not limited to the Examples without departing from the scope of the Claims.

All the parts and percentages are on the basis of weight, unless otherwise specified.

Example 1

For the purpose of examining the antimicrobial and deodorant properties, the function of promoting the growth of farm products, and the unelusive property, six compositions A to F and a comparative composition G, listed in Table 1, were prepared.

For preparation of the composition A, 6 parts by weight of silver nitride (c) and 50 parts of hot water (d) (at about 60 °C) were supplied into a preparation vessel and lightly stirred for dissolution of the silver nitride.

Then, to the water solution, 200 parts of water (d), 40 parts of zeolite (b), and 40 parts of ultrafine granular silica were added with stirring. After about 3 hours of a rest, 392 parts of isopropanol (e), 20 parts of potassium titanate whiskers (b), and 7.50 parts of methyltrimethoxysilane (a) were added and stirred at a speed of 1000 rpm for about 10 minutes.

After 5-hour maturing of the resultant liquid mixture, the composition A was obtained.

For preparation of the composition B, 2 parts of copper nitride (c) and 300 parts of silica were supplied into a preparation vessel and lightly stirred. Then, 80 parts of activated carbon was added and also, stirred. 293 parts of isopropanol (e), 100 parts of butylsellosolve, and 220 parts of methyltrimethoxysilane (a) were added and stirred at a speed of 1,000 rpm for about 10 minutes. After 5-hour maturing of the resultant liquid mixture, the composition B was obtained. Also, the other compositions C to F were prepared by similar procedures.

T A B L E  1

| | Composition | | | | | | |
|---|---|---|---|---|---|---|---|
| | A | B | C | D | E | F | G |
| **Components (part)** | | | | | | | |
| (A) Methy trimethoxy silane | 250 | 220 | 120 | 180 | 200 | – | 220 |
| Methy triethoxy silane | – | – | 120 | – | – | 250 | – |
| Tetraethoxy silane | – | – | – | – | 100 | – | – |
| (B) Silicagel | – | – | 50 | – | 100 | – | 40 |
| Zeolite | 40 | – | – | 50 | – | 40 | 40 |
| Activated carbon | – | 80 | 50 | – | – | – | – |
| Ultrafine silica | 40 | – | – | – | – | 40 | – |
| Colloidal silica | – | 300 | – | – | – | – | – |
| Colloidal alumina | – | – | – | 400 | – | – | – |
| Potassium titanate – whisker | 20 | – | – | – | 30 | 20 | – |
| (C) Caustic silver | 6 | – | 3 | – | 5 | 6 | 0.2 |
| Copper nitrate | – | 2 | – | – | 5 | – | – |
| Zinc nitrate | – | – | 1 | – | – | – | – |
| Copper bromide | – | – | – | 20 | – | – | – |
| (D) Water | 250 | – | 150 | – | 200 | 250 | 220 |
| (E) Isopropanol | 392 | 293 | 344 | 350 | 100 | 392 | 229.8 |
| Butylcellosolve | – | 100 | – | – | 260 | – | – |
| White pigment | – | – | 150 | – | – | – | – |
| Green pigment | – | – | – | – | – | – | 250 |
| **TOTAL** | 1,000 | 1,000 | 1,000 | 1,000 | 1,000 | 1,000 | 1,000 |

Then, the foregoing compositions were applied onto and coated over granular materials which had been prepared from particles of silica sand, river sand, rubble stone, and baked clay and beads of glass, for preparation of test antimicrobial deodorants.

The number, size, apparent specific gravity, components, coating amount, and curing process of each test piece are described in Table 2.

8

T A B L E  2

| Test piece No. | | Granular material | | | Composi-tion | Application of composition and curing |
|---|---|---|---|---|---|---|
| | | Description | Average diameter (mm) | Apparent specific gravity (g/cm³) | | |
| 1 | ( Example ) | Silica sand | 0.8 | 2.4 | A | 20g/1kg,250°C/30min. |
| 2 | ( Example ) | Silica sand | 0.8 | 2.4 | B | 20g/1kg,250°C/30min. |
| 3 | ( Example ) | Silica sand | 0.8 | 2.4 | C | 20g/1kg,250°C/30min. |
| 4 | (Comparative ex.) | Silica sand | 0.8 | 2.4 | G | 20g/1kg,250°C/30min. |
| 5 | ( Example ) | River sand | 1.5 | 2.3 | A | 18g/1kg,250°C/30min. |
| 6 | ( Example ) | River sand | 1.5 | 2.3 | D | 18g/1kg,250°C/30min. |
| 7 | ( Example ) | River sand | 1.5 | 2.3 | E | 18g/1kg,250°C/30min. |
| 8 | ( Example ) | River sand | 1.5 | 2.3 | F | 18g/1kg,250°C/30min. |
| 9 | ( Example ) | Rubble | 2 | 2.4 | B | 23g/1kg,300°C/20min. |
| 10 | ( Example ) | Rubble | 2 | 2.4 | C | 23g/1kg,300°C/20min. |
| 11 | ( Example ) | Rubble | 2 | 2.4 | D | 23g/1kg,300°C/20min. |
| 12 | (Comparative ex.) | Rubble | 2 | 2.4 | G | 23g/1kg,300°C/20min. |
| 13 | ( Example ) | Glass beads | 3 | 2.4 | A | 15g/1kg,200°C/40min. |
| 14 | ( Example ) | Glass beads | 3 | 2.4 | E | 15g/1kg,200°C/40min. |
| 15 | ( Example ) | Glass beads | 3 | 2.4 | F | 15g/1kg,200°C/40min. |
| 16 | (Comparative ex.) | Glass beads | 3 | 2.4 | G | 15g/1kg,200°C/40min. |
| 17 | ( Example ) | Baked clay | 10 | 0.8 | B | 30g/1kg,250°C/30min. |
| 18 | ( Example ) | Baked clay | 10 | 0.8 | C | 30g/1kg,250°C/30min. |
| 19 | ( Example ) | Baked clay | 10 | 0.8 | D | 30g/1kg,250°C/30min. |
| 20 | ( Example ) | Baked clay | 10 | 0.8 | F | 30g/1kg,250°C/30min. |

※ Application of composition is in g per a kg of granular material and curing is in °C/min.

Test 1

The antimicrobial test was conducted using the test pieces 1 to 4, 9 to 12, and 17 to 20.

(Test outline)

In the ion exchange water (referred to as a test liquid, hereinafter) to which 3 % (w/v) of each test piece was added, a microbial liquid carrying colitis or green pus germs was maintained at 30°C and at the end of a 6-hour period, the number of living germs was counted.

1) Preparation of microbial liquid

The culture solution containing test bacteria cultivated at 35°C in a common bouillon culture medium for one night was directly diluted with a sterilized physiologic saline so that the number of bacteria was about $10^7$ for 1 milliliter.

2) Preparation of test liquid

The test liquid was prepared by adding 3 % (w/v) of each test piece into a sterilized ion exchange water. Also, another sterilized ion water containing no test piece was prepared for reference.

3) Test procedure

1 ml of the microbial liquid was added to 100 ml of the test liquid and the mixed liquid was maintained at 30°C.

4) Measurement of number of bacteria

After 6 hours of a rest, the number of bacteria was measured by means of a mixed plaque culture method employing a standard agar culture medium. The cultivation at 35°C took place for 48 hours. The test results (the number of living bacteria in 1 ml of the test liquid) are shown in Table 3. As shown in Table 3, the test liquid 1 contained the test piece 1, the test liquid 2 contained the test piece 2, and so on.

## T A B L E 3

| Test bacteria | Colon bacillus | Bacillus of green pus |
|---|---|---|
| Number of theoretical – additive bacteria | $9.8 \times 10^4$ | $2.2 \times 10^5$ |
| Test liquid 1 ( Example ) | less than 1 | less than 1 |
| Test liquid 2 ( Example ) | less than 1 | $3.5 \times 10^2$ |
| Test liquid 3 ( Example ) | less than 1 | less than 1 |
| Test liquid 4 (Comparative ex.) | $6.8 \times 10^3$ | $7.1 \times 10^4$ |
| Test liquid 9 ( Example ) | less than 1 | $5.4 \times 10^2$ |
| Test liquid 10 ( Example ) | less than 1 | less than 1 |
| Test liquid 11 ( Example ) | less than 1 | less than 1 |
| Test liquid 12 (Comparative ex.) | $2.5 \times 10^4$ | $4.8 \times 10^4$ |
| Test liquid 17 ( Example ) | less than 1 | $1.6 \times 10^2$ |
| Test liquid 18 ( Example ) | less than 1 | less than 1 |
| Test liquid 19 ( Example ) | less than 1 | less than 1 |
| Test liquid 20 ( Example ) | less than 1 | less than 1 |
| Ion exchange water | $1.6 \times 10^4$ | $8.8 \times 10^4$ |

In Table 3, "less than 1" means no detection of bacteria due to the operational limit of the measurement method employed in this Test.

Test 2

The antifungus test was conducted using two of the test pieces 5 and 16 by the following manner.

(Test outline)

After the implantation and cultivation of test bacteria, which had been provided in a liquid, in a liquid culture medium which contained a given concentration amount of each test piece, the least concentration for inhibiting the growth of the bacteria was measured as a minimum growth inhibiting concentration.

1) Test bacteria

Three kinds of test bacteria were provided; apergillus niger IFO 4407, cladosporium cladostorioides IFO 6348, and penicillium citrinum IFO 7784.

2) Preparation of test microbial liquid

The test bacteria listed above were implanted into a proliferating culture medium (of agar base) and

EP 0 459 003 A1

after the cultivation at 25°C for 10 days, the resultant spores (or conidiospores) were added into a sterilized, 0.005% slufosuccinic acid dioctyl sodium solution so that the suspension thereof was about $10^6$/ml.

3) Cultivation

0.1 ml of the microbial liquid was added to a sensitive measurement culture medium (prepared by adding a given amount of the dried, sterilized test pieces to 10 ml of a culture medium for measurement) and then, the cultivation was carried out, with shaking, at 25°C for 5 to 7 days.

4) Determination

After the cultivation, the least concentration which inhibited the growth of bacteria was regarded as a minimum growth inhibiting concentration. The test results are shown in Tables 4-1 and 4-2.

T A B L E  4 - 1  ( Test piece 5, Example )

| Additive Concentration of the piece 5 (%) | Test bacteria | | |
|---|---|---|---|
| | Aspergillus niger IFO 4407 | Cladpsporium clad-Osporioides IFO 6348 | Penicillium citrunum IFO 7784 |
| 2 | − | − | − |
| 1 | − | − | − |
| 0.5 | − | − | − |
| 0.25 | − | − | − |
| 0.125 | − | − | − |
| 0.06 | − | − | − |
| 0.03 | − | − | − |
| 0.02 | + | + | + |
| 0.01 | + | + | + |

T A B L E  4 - 2  ( Test piece 16, Comparative ex. )

| Additive Concentration of the piece 16 (%) | Test bacteria | | |
|---|---|---|---|
| | Aspergillus niger IFO 4407 | Cladpsporium CLAD-Osporioides IFO 6348 | Penicillium citrunum IFO 7784 |
| 2 | + | + | + |
| 1 | + | + | + |
| 0.5 | + | + | + |
| 0.25 | + | + | + |
| 0.125 | + | + | + |
| 0.06 | + | + | + |
| 0.03 | + | + | + |
| 0.02 | + | + | + |
| 0.01 | + | + | + |

※ + represents growth of bacteria and − indicates no generation of bacteria

Test 3

For examining the antimicrobial activity to river water, the antimicrobial test was conducted using the test pieces 1 and 6.

11

(Test outline)

3 %, 5 %, and 10 % by volumetic weight of the test piece were added to river waters respectively. The test for change with time of colitis germs and general bacteria was carried out with respect to two conditions of stationary rest and continuous stirring.

1) Analysis test

This test was conducted conforming to a Test for service water (described in Japanese Environment Office, Publication 59, 1971). The lower limit was determined at 0 colitis germ for 100 ml and for general bacteria, 0 per 1 ml.

2) Reference water

Another river water (which contained non of the test piece) was provided as the reference water. The test results are shown in Tables 5-1 and 5-2.

## T A B L E  5 - 1  ( Example )

|  |  |  | Colon bacillus ( MPN/100ml ) | General bacteria ( No./ ml ) |
|---|---|---|---|---|
| Continuous stirring | 3% addition | 1 day | 0 | 11 |
|  |  | 6 days | 0 | 4 |
|  |  | 24 days | 0 | 2 |
|  | 5% addition | 1 day | 0 | 2 |
|  |  | 6 days | 0 | 6 |
|  |  | 24 days | 0 | 3 |
|  | 10% addition | 1 day | 0 | 2 |
|  |  | 6 days | 0 | 11 |
|  |  | 24 days | 0 | 2 |
|  | reference   water |  | 7,900 | 2,400 |

12

T A B L E  5 - 2  ( Example )

|  |  |  | Colon bacillus ( MPN/100ml ) | General bacteria ( No./ ml ) |
|---|---|---|---|---|
| Stationary | 3% addition | 6 days | 0 | 6 |
|  |  | 24 days | 0 | 12 |
|  |  | 48 days | 0 | 7 |
|  | 5% addition | 6 days | 0 | 4 |
|  |  | 24 days | 0 | 23 |
|  |  | 48 days | 0 | 10 |
|  | 10% addition | 6 days | 0 | 6 |
|  |  | 24 days | 0 | 3 |
|  |  | 48 days | 0 | 3 |
|  | reference  water | | 7,900 | 2,400 |

Test 4

For examining the presence of elusion of metal ions, the analysis test was conducted by the following procedure using the test pieces 13 and 14.

(Test outline)

This analysis test for comparison between a test water prepared by immersing the test piece into service water and a reference service water was carried out conforming to the regulations for quality control of service water (stipulated by Japanese Ministry of Health and Welfare, Ministerial Ordinance No.56, 1978).

1) Preparation of test water

200 g of the test piece was immersed into 4 liters of service water and maintained at a room temperature. After 24 hours, the supernatant liquid was recovered.

2) Reference water

The service water utilized for preparation of the test water was maintained under the same conditions and after 24 hours, the entire amount was recovered.
The test results are shown in Table 6.

EP 0 459 003 A1

T A B L E  6 ( Example )

| Detection item | Test piece 14 immersion water | Test piece 14 immersion water | Service water |
|---|---|---|---|
| Nitratenitrogen and Nitrite nitrogen | 1.5 mg/l. | 1.5 mg/l. | 1.5 mg/l. |
| Chlorine | 10 mg/l. | 10 mg/l. | 10 mg/l. |
| Organic ion (consumption of potassium permanganate) | 1.5 mg/l. | 1.7 mg/l. | 1.5 mg/l. |
| General bacteria | less than 30/ml. | less than 30/ml. | less than 30/ml. |
| Colon bacillus group | Undetected | Undetected | Undetected |
| Cyanogen ion | Undetected | Undetected | Undetected |
| Mercury | Undetected | Undetected | Undetected |
| Organic phosphorus | Undetected | Undetected | Undetected |
| Copper | below 0.01mg/l. | below 0.01mg/l. | below 0.01mg/l. |
| Iron | below 0.05mg/l. | below 0.05mg/l. | below 0.05mg/l. |
| Manganese | below 0.01mg/l. | below 0.01mg/l. | below 0.01mg/l. |
| Zinc | 0.008mg/l. | 0.008mg/l. | 0.008mg/l. |
| Lead | below 0.01mg/l. | below 0.01mg/l. | below 0.01mg/l. |
| Sexivalent chromium | below 0.02mg/l. | below 0.02mg/l. | below 0.02mg/l. |
| Cadmium | below 0.005mg/l. | below 0.005mg/l. | below 0.005mg/l. |
| Arsenic | below 0.005mg/l. | below 0.005mg/l. | below 0.005mg/l. |
| Fluorine | below 0.15mg/l. | below 0.15mg/l. | below 0.15mg/l. |
| Calcium, Magnesium | 52 mg/l. | 52 mg/l. | 52 mg/l. |
| Residue on evaporation | 110mg/l. | 110mg/l. | 110mg/l. |
| Varieties of phenol | below 0.005mg/l. | below 0.005mg/l. | below 0.005mg/l. |
| Anionic surfactant | less than 0.2mg/l. | less than 0.2mg/l. | less than 0.2mg/l. |
| PH scale | 7.2 | 7.2 | 7.2 |
| Scent | Normal | Normal | Normal |
| Taste | Normal | Normal | Normal |
| Degree of unclearness | less than 1 degree | less than 1 degree | less than 1 degree |
| Silver | less than 0.01mg/l. | less than 0.01mg/l. | less than 0.01mg/l. |

Test 5

The deodorant test was conducted by the following procedure using the test pieces 8 and 18.

(Test outline)

Using specimens prepared by applying 10 % the test piece 8 and 10 % the test piece 18 to an odorful object and a reference specimen carrying non of the test piece, the organoleptic examination was carried out for measurement of the intensity of odors with time.

1) Preparation of odorful object

600g of beef and 600 g of tuna meat were placed 200 g each in six 3-liter glass bottles followed by each 200 cc of service water. After 25 days, the resultant odorful objects (original odor) were acquired.

2) Intensity of odor

The measurements were carried out by six examiners (4 males, and 2 females) according to the 6-level odor intensity scale depicted in an Environment Office Publication.

3) Specimen

The specimens are described in Table 7.

TABLE 7

| Specimen | Beef (g) | Tuna fish (g) | service water (CC) | Test piece | |
|---|---|---|---|---|---|
| | | | | Name | Amount of addition |
| 101 | 200 | - | 200 | 8 | 40g |
| 102 | 200 | - | 200 | 18 | 40g |
| 103 | 200 | - | 200 | Nonadditive | - |
| 104 | - | 200 | 200 | 8 | 40g |
| 105 | - | 200 | 200 | 18 | 40g |
| 106 | - | 200 | 200 | Nonadditive | - |

The test results are shown in Table 8.

TABLE 8

| | Specimen | | | | | |
|---|---|---|---|---|---|---|
| | 101 Example | 102 Example | 103 Compara- tive ex. | 104 Example | 105 Example | 106 Compara- tive ex. |
| Degree of original scent | 5 | 5 | 5 | 5 | 5 | 5 |
| Degree after 10 min. | 1 | 1 | 5 | 2 | 1 | 5 |
| Degree after 2 hours | 0 | 0 | 5 | 1 | 0 | 5 |
| Degree after 24 hours | 0 | 0 | 5 | 1 | 0 | 5 |
| Degree after 7 days | 0 | 0 | 5 | 1 | 0 | 5 |
| Degree after 30 days | 0 | 0 | 5 | 1 | 0 | 5 |

Test 6

For examining the growth of vegetables cultivated with no use of agricultural sterilizer, the experiment was conducted by growing spinach with the use of the test pieces 7 and 15.

(Experiment procedure)

300 g of the test piece 7 and 300 g of the test piece 15 were scattered over two cultivating fields A and B (10 square meters each) respectively which had been prepared in a vinyl-covered green house. A reference field C (10 m²) was also provided for comparing purpose.

Then, spinach was planted in the three fields and cultivated under the same conditions, e.g. equal amount of fertilizer, with no use of chemical sterilizer.

The date of planting and harvest, the resultant output, and the length and appearance of products are shown in Table 9.

EP 0 459 003 A1

T A B L E   9

| Cultivated land | Date of planting | Date of harvest | Amount of harvest |
|---|---|---|---|
| A ( Example ) | 1989.10.5 | 1989.11.10 | 24.8kg |
| B ( Example ) | 1989.10.5 | 1989.11.10 | 24.2kg |
| C (Comparative ex.) | 1989.10.5 | 1989.11.10 | 17.2kg |

T A B L E   9 (Cont.)

| Cultivated land | Height of grass | Appearance |
|---|---|---|
| A ( Example ) | 30~35 | lively with large leaves |
| B ( Example ) | 29~34 | lively with large leaves |
| C (Comparative ex.) | 21~26 | yellowish spots in leaves |

Test 7

For examining the growth of rice crops, the experiment was carried out by the following procedure using the test piece 5.

(Experiment procedure)

400 g of the test piece 5 was scattered (4 g for one square meter) over a cultivating field D (of about 100 m²), one of the two fields prepared in Niigata Prefecture, Japan, and the other field E (equal size) was provided for reference.

2,160 bunches of rice plant raised on a separate seed bed were transplanted in each field (72 bunches for 3.3 m²) on 25th of May, 1989 and cultivated under the same conditions, e.g. equal amounts of sterilizers, fertilizers, and water supply, prior to the harvesting on 20th of September, 1989. The output of rice crops is shown in Table 10.

T A B L E   10

| | D field (Example) | E field (Comparative ex.) |
|---|---|---|
| Harvest weight (kg) | 55.08 | 45.79 |
| Number of rice grains Per 200 stubbles | 169 | 127 |

Claims

1. A coating composition comprising:

(a) 5 to 45 parts by weight of at least a material which is selected from the group consisting of organoalkoxysilane represented by the general formula, $R^1Si(OR^2)_3$ (where $R^1$ is an organic group having 1 to 8 carbon atoms and $R^2$ is an acyl group having 1 to 4 carbon atoms), hydrolyzate of the alkoxysilane, and partial condensates thereof;

(b) 0.3 to 40 parts by weight of at least a filler which is selected from the group consisting of silica gel, zeolite, activated carbon, ultrafine metallic oxide, and potassium titanate whisker;

(c) 0.03 to 3 parts by weight of an antimicrobial metal comprising at least a metal salt which is selected from the group consisting of silver, copper, and zinc salts;

(d) 3 to 60 parts by weight of water; and

(e) 5 to 80 parts of an alcohol, (where (a) + (b) + (c) + (d) + (e) = 100 parts by weight).

16

2. An antimicrobial deodorant formed by coating a granular material with the coating composition according to Claim 1 which is in turn heated for curing.

3. An antimicrobial deodorant according to Claim 2, wherein the granular material is at least selected from the group consisting of particles of sand, rubble stone, shell, glass, and baked inorganic clay matter having a particle size of 0.1 to 30 mm in average diameter.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 112, no. 7, 12 February 1990 Columbus, Ohio, USA ICHIKAWA, Yoshio: "Bactericidal siloxane coating compositions and textiles coated therewith." page 126; ref. no. 58371U<br>* abstract * | 1-3 | C 09 D 183/04<br>C 09 D 5/14<br>A 61 L 9/01<br>A 01 N 59/16<br>A 01 N 25/08 |
| A | FR-A-2 601 684 (CENTRE TECHNIQUE DES TUILES ET BRIQUES)<br>* the whole document * | 1-3 | |
| A | PATENT ABSTRACTS OF JAPAN vol. 11, no. 184 (C-427)(2631) 12 June 1987,<br>& JP-A-62 007747 (KANEBO LTD.) 14 January 1987,<br>* the whole document * | 1-3 | |
| A | PATENT ABSTRACTS OF JAPAN vol. 12, no. 301 (C-521)(3148) 16 August 1988,<br>& JP-A-63 075073 (YOSHIO ICHIKAWA) 5 April 1988,<br>* the whole document * | 1 | |
| A | PATENT ABSTRACTS OF JAPAN vol. 11, no. 282 (C-446)(2729) 11 September 1987,<br>& JP-A-62 079274 (YOSHIO ICHIKAWA) 11 April 1987,<br>* the whole document * | 1 | **TECHNICAL FIELDS SEARCHED (Int. Cl.5)**<br><br>C 09 D<br>A 01 N<br>C 08 K<br>A 61 L<br>C 02 F<br>C 04 B |

The present search report has been drawn up for all claims

| Place of search | Date of completion of search | Examiner |
|---|---|---|
| The Hague | 04 February 91 | HOLLENDER C.J.F. |